# EUROPEAN PATENT SPECIFICATION

(11) **EP 4 529 842 B1**
(45) Date of publication and mention of the grant of the patent: **29.07.2026**
(21) Application number: 24196883.3
(22) Date of filing: 28.08.2024
(51) Int. Cl.: A61B 5/372, A61B 5/00

(54) **METHOD AND SYSTEM FOR REAL-TIME CALIBRATION OF EAR-EEG DEVICE**
VERFAHREN UND SYSTEM ZUR ECHTZEITKALIBRIERUNG EINER OHR-EEG-VORRICHTUNG
PROCÉDÉ ET SYSTÈME D'ÉTALONNAGE EN TEMPS RÉEL DE DISPOSITIF D'ÉCOUTEUR-BOUTON

(30) Priority: 27.09.2023 IN 202321065013
(43) Date of publication of application: 02.04.2025
(73) Proprietor: Tata Consultancy Services Limited, Maharashtra (IN)
(72) Inventor: GUBBI LAKSHMINARASIMHA, Jayavardhana Rama, 560066 Bangalore, Karnataka (IN); JAYAS, Tanuja, 560066 Bangalore, Karnataka (IN); MURALIDHARAN, Kartik, 560066 Bangalore, Karnataka (IN); ANAND, Adarsh, 560066 Bangalore, Karnataka (IN); RAMAKRISHNAN, Ramesh Kumar, 560066 Bangalore, Karnataka (IN); PAL, Arpam, 700135 Kolkata, West Bengal (IN)
(74) Representative: Goddar, Heinz J.

(56) References cited:
- CN-A- 116 439 714
- US-A1- 2018 116 514
- US-A1- 2019 320 979
- KIM MINJAE ET AL: "Miniaturization for wearable EEG systems: recording hardware and data processing", BIOMEDICAL ENGINEERING LETTERS, THE KOREAN SOCIETY OF MEDICAL AND BIOLOGICAL ENGINEERING, KOREA, vol. 12, no. 3, 6 June 2022 (2022-06-06), pages 239 - 250, XP037909806, ISSN: 2093-9868, [retrieved on 20220606], DOI: 10.1007/S13534-022-00232-0

## Description

### CROSS-REFERENCE TO RELATED APPLICATIONS AND PRIORITY

The present application claims priority from Indian patent application number 202321065013, filed on September 27, 2023.

### TECHNICAL FIELD

The disclosure herein generally relates to the field of a signal processing, and more particularly, a method and system for a real-time calibration of one or more bioelectric signals received from a wearable Ear-EEG device.

### BACKGROUND

Electroencephalography (EEG) is a widely used noninvasive technique for measuring the electrical activity of the brain. The EEG has extensive applications in the study of brain functions in various domains like neurophysiology, braincomputer interface (BCI) and neuro-marketing. Lack of mobility, discomfort and strong decoding methods are a few obstacles in the monitoring of the brain activity. The main requirement for the interpretation and application of an EEG is good signal quality. Traditional approaches of assessing EEG signal quality involve visual inspection of the EEG time series for assessing any change in signal features. Further, the analysis of EEG signals for everyday in-situ applications is hindered by many challenges including artifacts induced from physiological and environmental sources as well as anatomical factors.

The acquisition of EEG signals from the scalp is very discomforting and does not have freedom of movement. Further, the EEG recording using ear wearables is a new modality which is more user friendly when compared to the existing scalp EEG. But the EEG signals recorded from these devices typically contain a lot of noise. Also, the EEG signals from some of the channels may not even get recorded because of lack of good skin-electrode connectivity.

Ear wearables are emerging technology and there is a lack of understanding of the EEG signals. Due to the nature of the brain signals, a standard template is not available against which the acquired EEG signals can be compared. So, it is important to collect Ear-EEG signal and calibrate it to get the proper signal, which would actually be Ear-EEG. Thus, it is important to check the quality of the EEG signal before using it for further downstream analysis. Visual inspection of signal is done to determine the presence of any kind of noise and to check if the signal was captured or not. For a consumer device, this approach is infeasible. Also, the inspection may differ from one expert to another. There is a lack of any particular method that can be used to check the quality of signals in real time for wearables to ensure optimal signal quality which can be used for applications such as early detection of neurodegenerative diseases and BCI. There is no such method present that can be used for the real-time calibration of such wearable devices, to understand the quality of the EEG signals from the device.

US 2019/320979 A1 describes a method for determining a signal quality metric of a set of electroencephalography (EEG) signals received at a set of sensors at an ear canal region.

### SUMMARY

The object of the present invention is achieved through a method, system, and non-transitory machine.-readable information storage medium according to the independent claims.

Embodiments of the present disclosure present technological improvements as solutions to one or more of the above-mentioned technical problems recognized by the inventors in conventional systems. For example, in one embodiment, a method for a real-time calibration of one or more signals received from a wearable Ear-EEG device is provided. The processor-implemented method includes receiving, via an input/output interface, a multitude of bioelectric signals of a user from a plurality of electrodes within a wearable Ear-EEG device, wherein the plurality of electrodes includes a ground electrode and a reference electrode.

Further, the processor-implemented method comprises collecting a set of context specific features from a mobile application within a mobile device and determining a contact of each of the plurality of electrodes with the user in order to receive the multitude of bioelectric signals based on a set of impedance values, wherein the set of impedance values is an impedance between each of the plurality of electrodes and a skin-body interface of the user. Furthermore, the processor-implemented method comprises verifying the received multitude of bioelectric signals from the plurality of electrodes based on a flat channel detection technique to have a predefined optimum voltage level, wherein one or more electrodes of the plurality of electrodes are removed corresponding to the multitude of bioelectric signals having a predefined saturated voltage level using the flat channel detection technique. Further, the processor-implemented method comprises selecting a set of electrodes of the plurality of electrodes which receives a set of EEG signals based on one or more characteristics of the multitude of bioelectric signals and the collected set of context specific features, wherein the one or more characteristics of the multitude of bioelectric signals is at least one of (1) in time domain, or (2) in frequency domain, or (3) in both time and frequency domain.

Furthermore, the processor-implemented method comprises checking the selected set of electrodes of the plurality of electrodes to send an alert to the user to recalibrate the Ear-EEG device if the selected set of electrodes are lesser than a predefined minimum number, evaluating a quality index value of each of the received set of EEG signals based on a predefined threshold value for the mobile application using a pre-trained machine learning model, wherein one or more characteristics of the received multitude of bioelectric signals along with the set of application specific features are used to determine the signal quality of the received set of EEG signals, and classifying the selected set of electrodes based on the evaluated quality index value of the set of EEG signals and a set of artifacts of the set of EEG signals, wherein a continuous detection of the set of artifacts of the received EEG signals is carried out. Finally, calibrating again the ear-EEG device based on the evaluated quality index value of the set of EEG signals received from the selected one or more electrodes.

In another aspect, a system for real-time calibration of one or more EEG signals received from a wearable Ear-EEG device is provided. The system comprises a memory storing a plurality of instructions and one or more Input/Output (I/O) interfaces to receive a multitude of bioelectric signals of a user from a plurality of electrodes within a wearable Ear-EEG device, wherein the plurality of electrodes includes a ground electrode and a reference electrode. Further, the system comprises one or more hardware processors coupled to the memory via the one or more I/O interfaces, wherein the one or more hardware processors are configured by the instructions to execute a plurality of modules of the system.

Further, the system is configured to collect a set of context specific features from a mobile application within a mobile device and determine a contact of each of the plurality of electrodes with the user in order to receive the multitude of bioelectric signals based on a set of impedance values. Wherein the set of context specific features are based on an initial context from day-to-day activities of the user collected automatically from the wearable Ear-EEG device and the wherein the set of impedance values is an impedance between each of the plurality of electrodes and a skin-body interface of the user. The received multitude of bioelectric signals are verified from the plurality of electrodes based on a flat channel detection technique to have a predefined optimum voltage level, wherein one or more electrodes of the plurality of electrodes are removed corresponding to the multitude of bioelectric signals having a predefined saturated voltage level using the flat channel detection technique. Further, a set of electrodes of the plurality of electrodes which receives a set of EEG signals are selected based on one or more characteristics of the multitude of bioelectric signals and the collected set of context specific features, wherein the one or more characteristics of the multitude of bioelectric signals is at least one of (1) in time domain, or (2) in frequency domain, or (3) in both time and frequency domain.

Furthermore, the system is configured to check the selected set of electrodes of the plurality of electrodes to send an alert to the user to recalibrate the Ear-EEG device if the selected set of electrodes are lesser than a predefined minimum number. Further, the system is configured to evaluate a quality index value of each of the received set of EEG signals based on a predefined threshold value for the mobile application using a pre-trained machine learning model and the selected set of electrodes are classified based on the evaluated quality index value of the set of EEG signals and a set of artifacts of the set of EEG signals. A continuous detection of the set of artifacts of the received EEG signals is carried out, wherein one or more characteristics of the received multitude of bioelectric signals along with the set of application specific features are used to determine the signal quality of the received set of EEG signals. Finally, the ear-EEG device is calibrated again based on the evaluated quality index value of the set of EEG signals received from the selected one or more electrodes.

In yet another aspect, there are provided one or more non-transitory machine-readable information storage mediums comprising one or more instructions, which when executed by one or more hardware processors causes a method for a real-time calibration of one or more EEG signals received from a wearable Ear-EEG device is provided. Further, the processor-implemented method comprises collecting a set of context specific features from a mobile application within a mobile device and determining a contact of each of the plurality of electrodes with the user in order to receive the multitude of bioelectric signals based on a set of impedance values, wherein the set of impedance values is an impedance between each of the plurality of electrodes and a skin-body interface of the user. Furthermore, the processor-implemented method comprises verifying the received multitude of bioelectric signals from the plurality of electrodes based on a flat channel detection technique to have a predefined optimum voltage level, wherein one or more electrodes of the plurality of electrodes are removed corresponding to the multitude of bioelectric signals having a predefined saturated voltage level using the flat channel detection technique. Further, the processor-implemented method comprises selecting a set of electrodes of the plurality of electrodes which receives a set of EEG signals based on one or more characteristics of the multitude of bioelectric signals and the collected set of context specific features, wherein the one or more characteristics of the multitude of bioelectric signals is at least one of (1) in time domain, or (2) in frequency domain, or (3) in both time and frequency domain.

Furthermore, the processor-implemented method comprises checking the selected set of electrodes of the plurality of electrodes to send an alert to the user to recalibrate the Ear-EEG device if the selected set of electrodes are lesser than a predefined minimum number, evaluating a quality index value of each of the received set of EEG signals based on a predefined threshold value for the mobile application using a pre-trained machine learning model, wherein one or more characteristics of the received multitude of bioelectric signals along with the set of application specific features are used to determine the signal quality of the received set of EEG signals, and classifying the selected set of electrodes based on the evaluated quality index value of the set of EEG signals and a set of artifacts of the set of EEG signals, wherein a continuous detection of the set of artifacts of the received EEG signals is carried out. Finally, calibrating again the Ear-EEG device based on the evaluated quality index value of the set of EEG signals received from the selected one or more electrodes.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates a system for real-time calibration of one or more EEG signals received from a wearable Ear-EEG device, according to some embodiments of the present disclosure.
FIG. 2 is a functional block diagram to illustrate a system for real-time calibration of one or more EEG signals received from a wearable Ear-EEG device, according to some embodiments of the present disclosure.
FIGS. 3A and 3B (collectively referred as FIG. 3) are an exemplary flow diagram illustrating a processor-implemented method for real-time calibration of one or more EEG signals received from a wearable Ear-EEG device, according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Electroencephalography (EEG) is a widely used noninvasive technique for measuring the electrical activity of the brain. The EEG has extensive applications in the study of brain functions in various domains like neurophysiology, braincomputer interface (BCI) and neuro-marketing. Lack of mobility, discomfort and strong decoding methods are a few obstacles in the monitoring of the brain activity. An Ear-EEG is more user friendly and portable in comparison to the existing scalp EEG. This form of wearable EEG finds various use cases in everyday life for detection/monitoring sleep, stress, emotion, and cognition.

The main requirement for the interpretation and application of an EEG is good signal quality. However, there is a lack of research on this topic in relation to newer mobile EEG devices, in particular ear worn devices. Traditional approaches of assessing EEG signal quality involve visual inspection of the EEG time series for assessing any change in signal features. Analysis of EEG signals for everyday in-situ applications is hindered by many challenges including artifacts induced from physiological and environmental sources as well as anatomical factors.

Recent studies have proposed analytical frameworks for the assessment of scalp EEG quality and identification of artifacts using rule-based methods. These methods are typically used in an offline processing manner, employed before signal analysis when abundant signals are available. With the advent of wearable devices, it is important to build techniques that work on short term signals giving us the ability of intervention based on the signal quality. An Ear-EEG is a new class of wearable device that requires assessment and calibration with short term signals periodically. To support this new modality, a detailed study of scalp and Ear-EEG signal is conducted from the perspective of different measures as well as time duration. An algorithm is developed to identify the epochs with electrooculography (EOG) and electromyography (EMG) artifacts in the Ear-EEG using a set of metrics based on the characteristics of EEG. Thresholds of these metrics are determined by training on a dataset containing synchronous ear and scalp EEG with good results.

Embodiments herein provide a method and system for a real-time calibration of one or more EEG signals received from a wearable Ear-EEG device. The system is leveraging quality of signals in real time for wearables to provide optimal signals which can be used for BCI applications. Further, the system is able to detect sensors (electrodes) in the wearable device where the signal has not been collected because the contact was not established.

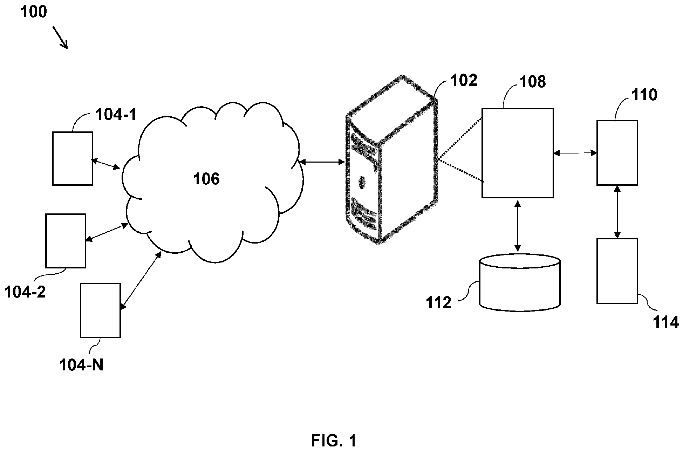

Referring now to the drawings, and more particularly to FIG. 1 through FIG. 3B, where similar reference characters denote corresponding features consistently throughout the figures, there are shown preferred embodiments, and these embodiments are described in the context of the following exemplary system and/or method.

FIG. 1 illustrates a block diagram of system for a real-time calibration of one or more EEG signals received from a wearable Ear-EEG device, in accordance with an example embodiment. Although the present disclosure is explained considering that the system 100 is implemented on a server, it may be understood that the system 100 may comprise one or more computing devices 102, such as a laptop computer, a desktop computer, a notebook, a workstation, a cloud-based computing environment and the like. It will be understood that the system 100 may be accessed through one or more input/output interfaces 104-1, 104-2... 104-N, collectively referred to as I/O interface 104. Examples of the I/O interface 104 may include, but are not limited to, a user interface, a portable computer, a personal digital assistant, a handheld device, a smartphone, a tablet computer, a workstation, and the like. The I/O interface 104 is communicatively coupled to the system 100 through a network 106.

In an embodiment, the network 106 may be a wireless or a wired network, or a combination thereof. In an example, the network 106 can be implemented as a computer network, as one of the different types of networks, such as virtual private network (VPN), intranet, local area network (LAN), wide area network (WAN), the internet, and such. The network 106 may either be a dedicated network or a shared network, which represents an association of the different types of networks that use a variety of protocols, for example, Hypertext Transfer Protocol (HTTP), Transmission Control Protocol/Internet Protocol (TCP/IP), and Wireless Application Protocol (WAP), to communicate with each other. Further, the network 106 may include a variety of network devices, including routers, bridges, servers, computing devices, storage devices. The network devices within the network 106 may interact with the system 100 through communication links.

The system 100 supports various connectivity options such as BLUETOOTH^{®}, USB, ZigBee, and other cellular services. The network environment enables connection of various components of the system 100 using any communication link including Internet, WAN, MAN, and so on. In an exemplary embodiment, the system 100 is implemented to operate as a stand-alone device. In another embodiment, the system 100 may be implemented to work as a loosely coupled device to a smart computing environment. Further, the system 100 comprises at least one memory 110 with a plurality of instructions, one or more databases 112, and one or more hardware processors 108 which are communicatively coupled with the at least one memory to execute a plurality of modules 114 therein. The components and functionalities of the system 100 are described further in detail.

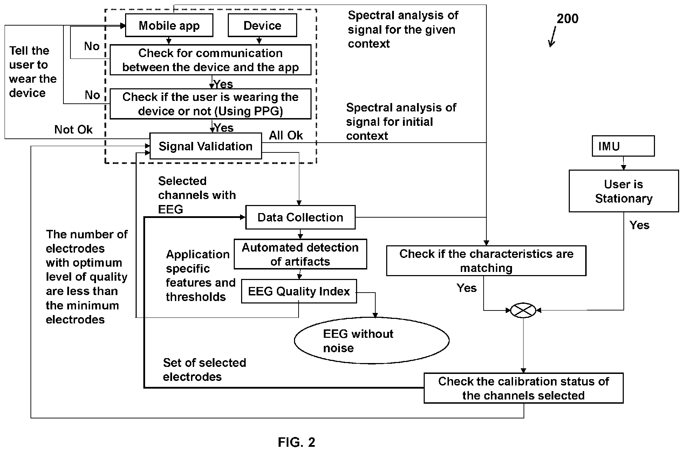

FIG. 2 is a functional block diagram 200 to illustrate the system 100 for real-time calibration of one or more signals received from a wearable Ear-EEG device, according to some embodiments of the present disclosure. In one embodiment, the system 100 is configured to measure a pair of impedance values between a plurality of electrodes and the human body to identify a set of electrodes which receives as well as do not receive the multitude of signals including EEG. Based on the pair of impedance values and a context from the day-to-day activities by a user, the set of electrodes where the EEG signal is being received is selected for signal extraction without disturbing the user. Further, the system 100 is configured to verify whether the voltage levels of the multitude of bioelectric signals received from the plurality of electrodes is attaining a saturation and based on the voltage level, those electrodes attaining saturation levels are removed from the plurality of electrodes.

In yet another embodiment, the system 100 is configured for selection of electrodes where the presence of EEG is determined using a set of context specific features extracted from a mobile application. The set of context specific features may vary according to the stimulus from activities of the user. Further, the system 100 is configured to estimate a quality index using a set of metrics based on characteristics of the EEG signal.

In another aspect, a framework is created to compute the quality metrics of an epoch and check if the metrics are in range of the thresholds of the EEG signal. The features evaluated in this section are statistical parameters which represent the complexity and strength of the Ear-EEG signal in both time and frequency domain. Further, the disclosure includes another embodiment for an artifact detection to identify and remove the artifacts in the Ear-EEG signals to get EEG signal in the end.

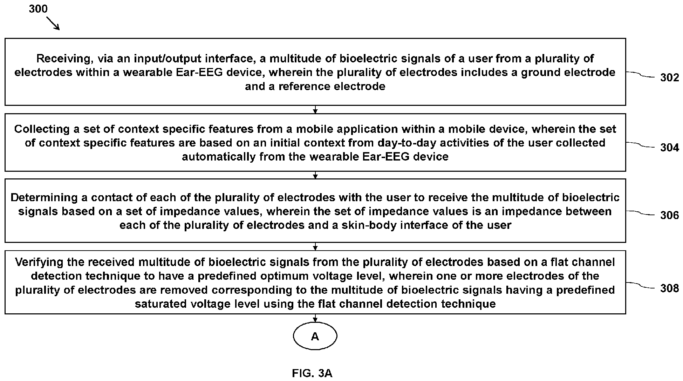

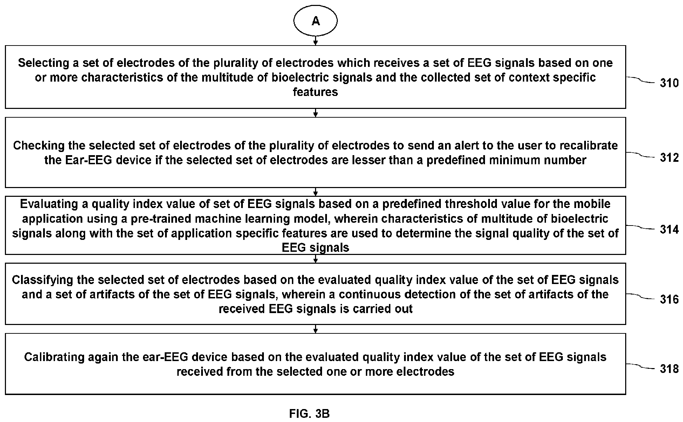

FIGS. 3A and 3B (collectively referred as FIG. 3) are a flow diagram illustrating a processor-implemented method 300 for a real-time calibration of one or more EEG signals received from a wearable Ear-EEG device implemented by the system 100 of FIG. 1. Functions of the components of the system 100 are now explained through steps of flow diagram in FIGS. 3A and 3B, according to some embodiments of the present disclosure.

Initially, at step 302 of the processor-implemented method 300, the one or more hardware processors 108 are configured by the programmed instructions to receive, via an input/output interface, a multitude of bioelectric signals of a user from a plurality of electrodes within a wearable Ear-EEG device. The plurality of electrodes includes a ground electrode and a reference electrode.

At the next step 304 of the processor-implemented method 300, the one or more hardware processors 108 are configured by the programmed instructions to collect a set of context specific features from a mobile application within a mobile device. The set of context specific features are based on an initial context from day-to-day activities of the user collected automatically from the wearable Ear-EEG device. An alert to wear the Ear-EEG device is sent to the user in case of an absence of the prompt message from the mobile application.

At the next step 306 of the processor-implemented method 300, the one or more hardware processors 108 are configured by the programmed instructions to determine a contact of each of the plurality of electrodes with the user in order to receive the multitude of bioelectric signals based on a set of impedance values. The set of impedance values is an impedance between each of the electrodes and skin-body interface of the user. The set of impedance values between the user (skin-body) and each of the plurality of electrodes should be a minimum (low impedance conductive path) compared to a predefined threshold value of impedance, to receive the multitude of bioelectric signals. A set of electrodes with low impedance values are selected to receive the multitude of bioelectric signals based on the set of impedance values measured.

At the next step 308 of the processor-implemented method 300, the one or more hardware processors 108 are configured by the programmed instructions to verify the received multitude of bioelectric signals from the plurality of electrodes based on a flat channel detection technique to have a predefined optimum voltage level. The one or more electrodes of the plurality of electrodes are removed corresponding to the multitude of bioelectric signals having a predefined saturated voltage level using the flat channel detection technique. The received multitude of bioelectric signals within the optimum voltage levels are collected for processing e.g., the amplitude of the signal should be between +/- 50 micro volts. The received multitude of bioelectric signals with the saturated voltage level +/- 500 micro volts are not actual EEG and hence rejected using the flat channel detection process.

At the next step 310 of the processor-implemented method 300, the one or more hardware processors 108 are configured by the programmed instructions to select a set of electrodes of the plurality of electrodes which receives a set of EEG signals based on one or more characteristics of the multitude of bioelectric signals and the collected set of context specific features. The one or more characteristics of the multitude of bioelectric signals is at least one of (1) in time domain, or (2) in frequency domain, or (3) in both time and frequency domain. The channel selection technique determines whether the received multitude of bioelectric signals are EEG or not. The selected set of EEG signals are separated from the received multitude of bioelectric signals using a predefined threshold voltage level based on a spatial characteristic (location of the electrode in the ear including concha or ear canal) as well as context specific features (the activity being performed by the user such as watching video, listening to music, playing a video game, etc.).

At the next step 312 of the processor-implemented method 300, the one or more hardware processors 108 are configured by the programmed instructions to check the selected set of electrodes of the plurality of electrodes to send an alert to the user to recalibrate the Ear-EEG device if the selected set of electrodes are lesser than a predefined minimum number. For a real-time check of the set of electrodes collecting the set of continuous EEG signal with quality levels for the specified application, a continuous check on the spectral characteristics of the set of EEG signals for the given context (Listening to music, watching a video, playing game) is done. Wherein, if the set of electrodes which receives the set of EEG signals are minimum, a user alert is sent to recalibrate the Ear-EEG device.

The status of the electrodes where the signal is being collected is checked when the spectral characteristics of the EEG signal for the given context is similar to the spectral characteristics of the initial context and at the same time the user is stationary. The information about the context and physical activity of the user is being collected from a sensor array (e.g., Inertial Measurement Unit (IMU)), wherein if the minimum number of electrodes checked when the user is stationary is achieved, the signal collection is continued if the number of electrodes collecting EEG is more than the required minimum number.

At the next step 314 of the processor-implemented method 300, the one or more hardware processors 108 are configured by the programmed instructions to evaluate a quality index value of each of the received set of EEG signals based on a predefined threshold value for the mobile application using a pre-trained machine learning model. The one or more characteristics of the received multitude of bioelectric signals along with the set of application specific features are used to determine the signal quality of the received set of EEG signals. Herein, a set of quality metrics based on characteristics of the received EEG signal is defined to measure the quality of the signal. The quality metric consists of features like: Signal-to-noise ratio (SNR), Root mean square (RMS) of the amplitude, Kurtosis of the amplitude, Skewness of the amplitude, mean of the power, zero crossing rate (ZCR), Spectral entropy, Maximum gradient, Auto-correlation function (ACF). The values of these metric will reflect the characteristics of the signal in both frequency and time domain.

In another aspect, a framework is created to compute the set of quality metrics for each of the set of EEG signal at an instant and check if the set of metrics are in range of a threshold. The thresholds for the quality index are application specific. The computed set of quality metrics is based on characteristics of the selected EEG signal defined to measure the quality of the signal. The set of quality metrics are constructed based on features which represent the complexity and strength of the set of EEG signals in both time and frequency domain. A spatial characteristic of the received multitude of bioelectric signals along with the set of context specific features are used to determine a signal quality of the received multitude of bioelectric signals. The signal quality varies according to the set of context specific features.

At the next step 316 of the processor-implemented method 300, the one or more hardware processors 108 are configured by the programmed instructions to classify the selected set of electrodes based on the evaluated quality index value of the set of EEG signals and a set of artifacts of the set of EEG signals, whether the set of artifacts (such as EOG and EMG) are present in them or not. Wherein a continuous detection of artifacts in the EEG signal collected is carried out and along with the quality of EEG signal is checked using an EEG Quality Index (EQI). The set of EEG signals are labelled as with the set of artifacts and without artifacts using machine learning algorithms. Tree based machine learning algorithms such as Decision Tree, Random Forest, Gradient Boost, XG Boost, Light Gradient boosting, are trained to classify the EEG signals. The defined metrics are calculated for a given segment of the EEG signals in four different bands of the signal namely, alpha, beta, gamma, and delta and for the raw signal. The evaluation of metrics in different bands captures the features of the signal in different spatial regions. This set of metrics for each segment is normalized and later is fed to a machine learning classifier which labels that particular segment with or without artifact.

Finally, at the last step 318 of the processor-implemented method 300, the one or more hardware processors 108 are configured by the programmed instructions to re-calibrate the ear-EEG device based on the evaluated quality index value of the set of EEG signals received from the selected one or more electrodes.

The written description describes the subject matter herein to enable any person skilled in the art to make and use the embodiments. The scope of the subject matter embodiments is defined by the claims.

The embodiments of present disclosure herein address unresolved problems of quality of signals in real time for wearables to provide optimal signals which can be used for BCI applications. Further, there is no such method present that can be used for the real-time calibration of wearable devices to understand the quality of the EEG signals from the wearable device. Embodiments herein provide a method and system for a real-time calibration of one or more EEG signals received from a wearable Ear-EEG device. The system is leveraging quality of signal in real time for wearables to provide optimal signals which can be used for BCI applications. Further, the system is able to detect sensors (electrodes) in the wearable where the signal has not been collected because the contact was not established.

This disclosure also comprises a program and in addition computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an applicationspecific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computerusable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor-implemented method (300) comprising:
receiving (302), via an Input/Output (I/O) interface, a multitude of bioelectric signals of a user from a plurality of electrodes within a wearable Ear-Electroencephalography (EEG) device, wherein the plurality of electrodes includes a ground electrode and a reference electrode;
collecting (304), via one or more hardware processors, a set of context specific features from a mobile application within a mobile device, wherein the set of context specific features are based on an initial context from day-to-day activities of the user collected automatically from the wearable Ear-EEG device, wherein the initial context is a prompt message from the mobile application to the wearable Ear-EEG device via the mobile device, the context relating to the activity being performed by the user;
determining (306), via the one or more hardware processors, a contact of each of the plurality of electrodes with the user in order to receive the multitude of bioelectric signals based on a set of impedance values, wherein the set of impedance values is an impedance between each of the plurality of electrodes and a skin-body interface of the user, and select a set of electrodes with low impedance compared to a predefined threshold in order to receive the multitude of bioelectric signals;
verifying (308), via the one or more hardware processors, the received multitude of bioelectric signals from the plurality of electrodes based on a flat channel detection technique to have a predefined optimum voltage level, wherein one or more electrodes of the plurality of electrodes are removed pertaining to the multitude of bioelectric signals having a predefined saturated voltage level using the flat channel detection technique;
selecting (310), via the one or more hardware processors, a set of electrodes of the plurality of electrodes which receives a set of EEG signals based on one or more characteristics of the multitude of bioelectric signals and the collected set of context specific features, wherein the one or more characteristics of the multitude of bioelectric signals is at least one of (1) in time domain, or (2) in frequency domain, or (3) in both time and frequency domain, wherein the selection technique determines whether the received multitude of bioelectric signals are EEG or not and the selected set of EEG signals are separated from the received multitude of bioelectric signals using a predefined threshold voltage level based on the characteristics and the context specific features;
checking (312), via the one or more hardware processors, the selected set of electrodes of the plurality of electrodes to send an alert to the user to recalibrate the Ear-EEG device if the selected set of electrodes is lesser than a predefined minimum number of electrodes;
evaluating (314), via the one or more hardware processors, a quality index value of each of the received set of EEG signals based on a predefined threshold value for the mobile application using a pre-trained machine learning model, wherein one or more characteristics of the received multitude of bioelectric signals along with the set of context specific features are used to determine the signal quality of the received set of EEG signals, wherein a set of quality metrics based on the characteristics of the received EEG signal is defined to measure the quality of the bioelectric signal, wherein the quality metrics comprise : Signal-to-noise ratio (SNR), Root mean square (RMS) of the amplitude, Kurtosis of the amplitude, Skewness of the amplitude, mean of the power, zero crossing rate (ZCR), Spectral entropy, Maximum gradient, Auto-correlation function (ACF), wherein values of the quality metrics reflect the characteristics of the bioelectric signal in frequency and time domain,
wherein the set of quality metrics is constructed based on features which represent complexity and strength of the set of EEG signals in time and/or frequency domain, wherein the characteristics of the received multitude of bioelectric signals along with the set of context specific features are used to determine the signal quality of the received multitude of bioelectric signals, wherein the signal quality varies according to the set of context specific features of the mobile application;
classifying (316), via the one or more hardware processors, the selected set of electrodes based on the evaluated quality index value of the set of EEG signals and a set of artifacts of the set of EEG signals, wherein a continuous detection of the set of artifacts of the received EEG signals is carried out, wherein the set of EEG signals are labelled as with the set of artifacts and without artifacts using machine learning algorithms, wherein the set of quality metrics for each segment is normalized and fed to a machine learning classifier which labels that particular segment with or without artifact; and
re-calibrating (318), via the one or more hardware processors, the ear-EEG device based on the evaluated quality index value of the set of EEG signals received from the selected one or more electrodes.

2. The processor-implemented method (300) as claimed in claim 1, wherein an alert to wear the Ear-EEG device is sent to the user in case of an absence of the prompt message from the mobile application.

3. The processor-implemented method (300) as claimed in claim 1, wherein a framework is created to compute a set of quality metrics for each of the selected one or more electrodes receiving the set of EEG signals at an instant and check if the set of quality metrics are in range of the predefined threshold value, and wherein the predefined threshold values for the quality index are an application specific.

4. The processor-implemented method (300) as claimed in claim 1, wherein:
if the quality index value drops below a predefined lowest threshold for the given mobile application by the user for a minimum number of electrodes, an EEG signal validation and an electrode selection are done again; and
if the quality index value is less than the predefined threshold value the electrode selection is carried out again.

5. A system (100) comprising:
a memory (110) storing instructions;
one or more Input/Output (I/O) interfaces (104); and
one or more hardware processors (108) coupled to the memory (110) via the one or more I/O interfaces (104), wherein the one or more hardware processors (108) are configured by the instructions to:
receive a multitude of bioelectric signals of a user from a plurality of electrodes within a wearable Ear-Electroencephalography (EEG) device, wherein the plurality of electrodes includes a ground electrode and a reference electrode;
collect a set of context specific features from a mobile application within a mobile device, wherein the set of context specific features are based on an initial context from day-to-day activities of the user collected automatically from the wearable Ear-EEG device, wherein the initial context is a prompt message from the mobile application to the wearable Ear-EEG device via the mobile device, the context relating to the activity being performed by the user;
determine a contact of each of the plurality of electrodes with the user in order to receive the multitude of bioelectric signals based on a set of impedance values, wherein the set of impedance values is an impedance between each of the plurality of electrodes and a skin-body interface of the user, , and select a set of electrodes with low impedance compared to a predefined threshold in order to receive the multitude of bioelectric signals;
verify the received multitude of bioelectric signals from the plurality of electrodes based on a flat channel detection technique to have a predefined optimum voltage level, wherein one or more electrodes of the plurality of electrodes are removed corresponding to the multitude of bioelectric signals having a predefined saturated voltage level using the flat channel detection technique;
select a set of electrodes of the plurality of electrodes which receives a set of EEG signals based on one or more characteristics of the multitude of bioelectric signals and the collected set of context specific features, wherein the one or more characteristics of the multitude of bioelectric signals is at least one of (1) in time domain, or (2) in frequency domain, or (3) in both time and frequency domain, wherein the selection technique determines whether the received multitude of bioelectric signals are EEG or not and the selected set of EEG signals are separated from the received multitude of bioelectric signals using a predefined threshold voltage level based on the characteristics and the context specific features;
check the selected set of electrodes of the plurality of electrodes to send an alert to the user to recalibrate the Ear-EEG device if the selected set of electrodes are lesser than a predefined minimum number of electrodes;
evaluate a quality index value of each of the received set of EEG signals based on a predefined threshold value for the mobile application using a pre-trained machine learning model, wherein one or more characteristics of the received multitude of bioelectric signals along with the set of context specific features are used to determine the signal quality of the received set of EEG signals, wherein a set of quality metrics based on the characteristics of the received EEG signal is defined to measure the quality of the bioelectric signal, wherein the quality metrics comprise: : Signal-to-noise ratio (SNR), Root mean square (RMS) of the amplitude, Kurtosis of the amplitude, Skewness of the amplitude, mean of the power, zero crossing rate (ZCR), Spectral entropy, Maximum gradient, Auto-correlation function (ACF), wherein values of the quality metrics reflect the characteristics of the bioelectric signal in frequency and time domain,
wherein the set of quality metrics is constructed based on features which represent complexity and strength of the set of EEG signals in time and/or frequency domain, wherein the characteristics of the received multitude of bioelectric signals along with the set of context specific features are used to determine the signal quality of the received multitude of bioelectric signals, wherein the signal quality varies according to the set of context specific features of the mobile application;
classify the selected set of electrodes based on the evaluated quality index value of the set of EEG signals and a set of artifacts of the set of EEG signals, wherein a continuous detection of the set of artifacts of the received EEG signals is carried out, wherein the set of EEG signals are labelled as with the set of artifacts and without artifacts using machine learning algorithms, wherein the set of quality metrics for each segment is normalized and fed to a machine learning classifier which labels that particular segment with or without artifact; and
re-calibrate the ear-EEG device based on the evaluated quality index value of the set of EEG signals received from the selected one or more electrodes.

6. The system (100) as claimed in claim 5, wherein an alert to wear the Ear-EEG device is sent to the user in case of an absence of the prompt message from the mobile application.

7. The system (100) as claimed in claim 5, wherein a framework is created to compute a set of quality metrics for each of the selected one or more electrodes receiving the set of EEG signals at an instant and check if the set of quality metrics are in range of the predefined threshold value, wherein the predefined threshold values for the quality index are an application specific.

8. The system (100) as claimed in claim 5, wherein:
if the quality index value drops below a predefined lowest threshold for the given mobile application by the user for a minimum number of electrodes, an EEG signal validation and an electrode selection are done again; and
if the quality index value is less than the predefined threshold value the electrode selection is carried out again.

9. One or more non-transitory machine-readable information storage mediums comprising one or more instructions which when executed by one or more hardware processors cause:
receiving, via an Input/Output (I/O) interface, a multitude of bioelectric signals of a user from a plurality of electrodes within a wearable Ear- Electroencephalography (EEG) device, wherein the plurality of electrodes includes a ground electrode and a reference electrode;
collecting a set of context specific features from a mobile application within a mobile device, wherein the set of context specific features are based on an initial context from day-to-day activities of the user collected automatically from the wearable Ear-EEG device and the initial context is a prompt message from the mobile application to the Ear-EEG device via the mobile device, and wherein an alert to wear the Ear-EEG device is sent to the user in case of an absence of the prompt message from the mobile application, wherein the initial context is a prompt message from the mobile application to the wearable Ear-EEG device via the mobile device, the context relating to the activity being performed by the user;
determining a contact of each of the plurality of electrodes with the user in order to receive the multitude of bioelectric signals based on a set of impedance values, wherein the set of impedance values is an impedance between each of the plurality of electrodes and a skin-body interface of the user and and selecting a set of electrodes with low impedance compared to a predefined threshold in order to receive the multitude of bioelectric signals;
verifying the received multitude of bioelectric signals from the plurality of electrodes based on a flat channel detection technique to have a predefined optimum voltage level, wherein one or more electrodes of the plurality of electrodes are removed pertaining to the multitude of bioelectric signals having a predefined saturated voltage level using the flat channel detection technique;
selecting a set of electrodes of the plurality of electrodes which receives a set of EEG signals based on one or more characteristics of the multitude of bioelectric signals and the collected set of context specific features, wherein the one or more characteristics of the multitude of bioelectric signals is at least one of (1) in time domain, or (2) in frequency domain, or (3) in both time and frequency domain, wherein the selection technique determines whether the received multitude of bioelectric signals are EEG or not and the selected set of EEG signals are separated from the received multitude of bioelectric signals using a predefined threshold voltage level based on the characteristics and the context specific features;
checking the selected set of electrodes of the plurality of electrodes to send an alert to the user to recalibrate the Ear-EEG device if the selected set of electrodes is lesser than a predefined minimum number of electrodes;
evaluating a quality index value of each of the received set of EEG signals based on a predefined threshold value for the mobile application using a pre-trained machine learning model, wherein one or more characteristics of the received multitude of bioelectric signals along with the set of context specific features are used to determine the signal quality of the received set of EEG signals, and the quality index value of each of the received set of EEG signals varies according to the context specific features of the mobile application, wherein a set of quality metrics based on the characteristics of the received EEG signal is defined to measure the quality of the bioelectric signal, wherein the quality metrics comprise : Signal-to-noise ratio (SNR), Root mean square (RMS) of the amplitude, Kurtosis of the amplitude, Skewness of the amplitude, mean of the power, zero crossing rate (ZCR), Spectral entropy, Maximum gradient, Auto-correlation function (ACF), wherein values of the quality metrics reflect the characteristics of the bioelectric signal in frequency and time domain,
wherein the set of quality metrics is constructed based on features which represent complexity and strength of the set of EEG signals in time and/or frequency domain, wherein the characteristics of the received multitude of bioelectric signals along with the set of context specific features are used to determine the signal quality of the received multitude of bioelectric signals, wherein the signal quality varies according to the set of context specific features of the mobile application;
classifying the selected set of electrodes based on the evaluated quality index value of the set of EEG signals and a set of artifacts of the set of EEG signals, wherein a continuous detection of the set of artifacts of the received EEG signals is carried out, wherein the set of EEG signals are labelled as with the set of artifacts and without artifacts using machine learning algorithms, wherein the set of quality metrics for each segment is normalized and fed to a machine learning classifier which labels that particular segment with or without artifact; and
re-calibrating the ear-EEG device based on the evaluated quality index value of the set of EEG signals received from the selected one or more electrodes.

10. The one or more non-transitory machine-readable information storage mediums of claim 9, wherein a framework is created to compute a set of quality metrics for each of the selected one or more electrodes receiving the set of EEG signals at an instant and check if the set of quality metrics are in range of the predefined threshold value, and wherein the predefined threshold values for the quality index are an application specific.

11. The one or more non-transitory machine-readable information storage mediums of claim 9, wherein:
if the quality index value drops below a predefined lowest threshold for the given mobile application by the user for a minimum number of electrodes, an EEG signal validation and an electrode selection are done again; and
if the quality index value is less than the predefined threshold value the electrode selection is carried out again.

## Patentansprüche

1. Prozessorimplementiertes Verfahren (300), umfassend:
Empfangen (302), über eine Eingabe/Ausgabe(I/O)-Schnittstelle, einer Vielzahl von bioelektrischen Signalen eines Benutzers von einer Mehrzahl von Elektroden innerhalb einer tragbaren Ohrenzephalographie(EEG)-Vorrichtung, wobei die Mehrzahl von Elektroden eine Masseelektrode und eine Referenzelektrode beinhaltet;
Sammeln (304), über einen oder mehrere Hardwareprozessoren, eines Satzes von kontextspezifischen Merkmalen von einer mobilen Anwendung innerhalb einer mobilen Vorrichtung, wobei der Satz von kontextspezifischen Merkmalen auf einem anfänglichen Kontext von täglichen Aktivitäten des Benutzers basiert, die automatisch von der tragbaren Ohren-EEG-Vorrichtung gesammelt werden, wobei der anfängliche Kontext eine Aufforderungsnachricht von der mobilen Anwendung an die tragbare Ohren-EEG-Vorrichtung über die mobile Vorrichtung ist, wobei sich der Kontext auf die Aktivität bezieht, die von dem Benutzer durchgeführt wird;
Bestimmen (306), über den einen oder die mehreren Hardwareprozessoren, eines Kontakts jeder der Mehrzahl von Elektroden mit dem Benutzer, um die Vielzahl von bioelektrischen Signalen basierend auf einem Satz von Impedanzwerten zu empfangen, wobei der Satz von Impedanzwerten eine Impedanz zwischen jeder der Mehrzahl von Elektroden und einer Haut-Körper-Schnittstelle des Benutzers ist, und Auswählen eines Satzes von Elektroden mit niedriger Impedanz im Vergleich zu einem vordefinierten Schwellenwert, um die Vielzahl von bioelektrischen Signalen zu empfangen;
Verifizieren (308), über den einen oder die mehreren Hardwareprozessoren, der empfangenen Vielzahl von bioelektrischen Signalen von der Mehrzahl von Elektroden basierend auf einer Flachkanaldetektionstechnik, um einen vordefinierten optimalen Spannungspegel aufzuweisen, wobei eine oder mehrere Elektroden der Mehrzahl von Elektroden, die zu der Vielzahl von bioelektrischen Signalen gehören, die einen vordefinierten gesättigten Spannungspegel aufweisen, unter Verwendung der Flachkanaldetektionstechnik entfernt werden;
Auswählen (310), über den einen oder die mehreren Hardwareprozessoren, eines Satzes von Elektroden der Mehrzahl von Elektroden, der einen Satz von EEG-Signalen basierend auf einer oder mehreren Eigenschaften der Vielzahl von bioelektrischen Signalen und dem gesammelten Satz von kontextspezifischen Merkmalen empfängt, wobei die eine oder die mehreren Eigenschaften der Vielzahl von bioelektrischen Signalen mindestens eines von (1) im Zeitbereich oder (2) im Frequenzbereich oder (3) sowohl im Zeit- als auch im Frequenzbereich ist, wobei die Auswahltechnik bestimmt, ob die empfangene Vielzahl von bioelektrischen Signalen EEG ist oder nicht, und der ausgewählte Satz von EEG-Signalen von der empfangenen Vielzahl von bioelektrischen Signalen unter Verwendung eines vordefinierten Schwellenspannungspegels basierend auf den Eigenschaften den kontextspezifischen Merkmalen getrennt wird;
Prüfen (312), über den einen oder die mehreren Hardwareprozessoren, des ausgewählten Satzes von Elektroden der Mehrzahl von Elektroden, um eine Warnung an den Benutzer zu senden, um die Ohren-EEG-Vorrichtung neu zu kalibrieren, wenn der ausgewählte Satz von Elektroden kleiner als eine vordefinierte Mindestanzahl von Elektroden ist;
Bewerten (314), über den einen oder die mehreren Hardwareprozessoren, eines Qualitätsindexwerts jedes des empfangenen Satzes von EEG-Signalen basierend auf einem vordefinierten Schwellenwert für die mobile Anwendung unter Verwendung eines vortrainierten maschinellen Lernmodells, wobei eine oder mehrere Eigenschaften der empfangenen Vielzahl von bioelektrischen Signalen zusammen mit dem Satz von kontextspezifischen Merkmalen verwendet werden, um die Signalqualität des empfangenen Satzes von EEG-Signalen zu bestimmen, wobei ein Satz von Qualitätsmetriken basierend auf den Eigenschaften des empfangenen EEG-Signals definiert ist, um die Qualität des bioelektrischen Signals zu messen, wobei die Qualitätsmetriken Folgendes umfassen: Signal-Rausch-Verhältnis (SNR), quadratisches Mittel (RMS) der Amplitude, Kurtosis der Amplitude, Schiefe der Amplitude, Mittelwert der Leistung, Nulldurchgangsrate (ZCR), spektrale Entropie, maximaler Gradient, Autokorrelationsfunktion (ACF), wobei Werte der Qualitätsmetriken die Eigenschaften des bioelektrischen Signals im Frequenz- und Zeitbereich widerspiegeln,
wobei der Satz von Qualitätsmetriken basierend auf Merkmalen konstruiert wird, die die Komplexität und Stärke des Satzes von EEG-Signalen im Zeit- und/oder Frequenzbereich darstellen, wobei die Eigenschaften der empfangenen Vielzahl von bioelektrischen Signalen zusammen mit dem Satz von kontextspezifischen Merkmalen verwendet werden, um die Signalqualität der empfangenen Vielzahl von bioelektrischen Signalen zu bestimmen, wobei die Signalqualität gemäß dem Satz von kontextspezifischen Merkmalen der mobilen Anwendung variiert;
Klassifizieren (316), über den einen oder die mehreren Hardwareprozessoren, des ausgewählten Satzes von Elektroden basierend auf dem bewerteten Qualitätsindexwert des Satzes von EEG-Signalen und einem Satz von Artefakten des Satzes von EEG-Signalen, wobei eine kontinuierliche Detektion des Satzes von Artefakten der empfangenen EEG-Signale durchgeführt wird, wobei der Satz von EEG-Signalen als mit dem Satz von Artefakten und ohne Artefakte unter Verwendung von maschinellen Lernalgorithmen gekennzeichnet wird, wobei der Satz von Qualitätsmetriken für jedes Segment normalisiert und einem maschinellen Lernklassifikator zugeführt wird, der dieses bestimmte Segment mit oder ohne Artefakt kennzeichnet; und
Neukalibrieren (318), über den einen oder die mehreren Hardwareprozessoren, der Ohren-EEG-Vorrichtung basierend auf dem bewerteten Qualitätsindexwert des Satzes von EEG-Signalen, die von der ausgewählten einen oder den ausgewählten mehreren Elektroden empfangen werden.

2. Prozessorimplementiertes Verfahren (300) nach Anspruch 1, eine Warnung zum Tragen der Ohren-EEG-Vorrichtung an den Benutzer gesendet wird, falls die Aufforderungsnachricht von der mobilen Anwendung nicht vorhanden ist.

3. Prozessorimplementiertes Verfahren (300) nach Anspruch 1, wobei ein Framework erstellt wird, um einen Satz von Qualitätsmetriken für jede der ausgewählten einen oder mehreren Elektroden zu berechnen, die den Satz von EEG-Signalen zu einem Zeitpunkt empfangen, und zu prüfen, ob der Satz von Qualitätsmetriken im Bereich des vordefinierten Schwellenwerts liegt, und wobei die vordefinierten Schwellenwerte für den Qualitätsindex anwendungsspezifisch sind.

4. Prozessorimplementiertes Verfahren (300) nach Anspruch 1, wobei:
wenn der Qualitätsindexwert unter einen vordefinierten niedrigsten Schwellenwert für die gegebene mobile Anwendung durch den Benutzer für eine Mindestanzahl von Elektroden fällt, eine EEG-Signalvalidierung und eine Elektrodenauswahl erneut durchgeführt werden; und
wenn der Qualitätsindexwert kleiner als der vordefinierte Schwellenwert ist, die Elektrodenauswahl erneut durchgeführt wird.

5. System (100), umfassend:
einen Speicher (110), der Anweisungen speichert;
eine oder mehrere Eingabe/Ausgabe(I/O)-Schnittstellen (104); und
einen oder mehrere Hardwareprozessoren (108), die mit dem Speicher (110) über die eine oder die mehreren I/O-Schnittstellen (104) gekoppelt sind, wobei der eine oder die mehreren Hardwareprozessoren (108) durch die Anweisungen konfiguriert sind zum:
Empfangen einer Vielzahl von bioelektrischen Signalen eines Benutzers von einer Mehrzahl von Elektroden innerhalb einer tragbaren Ohrenzephalographie(EEG)-Vorrichtung, wobei die Mehrzahl von Elektroden eine Masseelektrode und eine Referenzelektrode beinhaltet;
Sammeln eines Satzes von kontextspezifischen Merkmalen von einer mobilen Anwendung innerhalb einer mobilen Vorrichtung, wobei der Satz von kontextspezifischen Merkmalen auf einem anfänglichen Kontext von täglichen Aktivitäten des Benutzers basiert, die automatisch von der tragbaren Ohren-EEG-Vorrichtung gesammelt werden, wobei der anfängliche Kontext eine Aufforderungsnachricht von der mobilen Anwendung an die tragbare Ohren-EEG-Vorrichtung über die mobile Vorrichtung ist, wobei sich der Kontext auf die Aktivität bezieht, die von dem Benutzer durchgeführt wird;
Bestimmen eines Kontakts jeder der Mehrzahl von Elektroden mit dem Benutzer, um die Vielzahl von bioelektrischen Signalen basierend auf einem Satz von Impedanzwerten zu empfangen, wobei der Satz von Impedanzwerten eine Impedanz zwischen jeder der Mehrzahl von Elektroden und einer Haut-Körper-Schnittstelle des Benutzers ist, und Auswählen eines Satzes von Elektroden mit niedriger Impedanz im Vergleich zu einem vordefinierten Schwellenwert, um die Vielzahl von bioelektrischen Signalen zu empfangen;
Verifizieren der empfangenen Vielzahl von bioelektrischen Signalen von der Mehrzahl von Elektroden basierend auf einer Flachkanaldetektionstechnik, um einen vordefinierten optimalen Spannungspegel aufzuweisen, wobei eine oder mehrere Elektroden der Mehrzahl von Elektroden entsprechend der Vielzahl von bioelektrischen Signalen, die einen vordefinierten gesättigten Spannungspegel aufweisen, unter Verwendung der Flachkanaldetektionstechnik entfernt werden;
Auswählen eines Satzes von Elektroden der Mehrzahl von Elektroden, der einen Satz von EEG-Signalen basierend auf einer oder mehreren Eigenschaften der Vielzahl von bioelektrischen Signalen und dem gesammelten Satz von kontextspezifischen Merkmalen empfängt, wobei die eine oder die mehreren Eigenschaften der Vielzahl von bioelektrischen Signalen mindestens eines von (1) im Zeitbereich oder (2) im Frequenzbereich oder (3) sowohl im Zeit- als auch im Frequenzbereich ist, wobei die Auswahltechnik bestimmt, ob die empfangene Vielzahl von bioelektrischen Signalen EEG ist oder nicht, und der ausgewählte Satz von EEG-Signalen von der empfangenen Vielzahl von bioelektrischen Signalen unter Verwendung eines vordefinierten Schwellenspannungspegels basierend auf den Eigenschaften und den kontextspezifischen Merkmalen getrennt wird;
Prüfen des ausgewählten Satzes von Elektroden der Mehrzahl von Elektroden, um eine Warnung an den Benutzer zu senden, um die Ohren-EEG-Vorrichtung neu zu kalibrieren, wenn der ausgewählte Satz von Elektroden kleiner als eine vordefinierte Mindestanzahl von Elektroden ist;
Bewerten eines Qualitätsindexwerts jedes des empfangenen Satzes von EEG-Signalen basierend auf einem vordefinierten Schwellenwert für die mobile Anwendung unter Verwendung eines vortrainierten maschinellen Lernmodells, wobei eine oder mehrere Eigenschaften der empfangenen Vielzahl von bioelektrischen Signalen zusammen mit dem Satz von kontextspezifischen Merkmalen verwendet werden, um die Signalqualität des empfangenen Satzes von EEG-Signalen zu bestimmen, wobei ein Satz von Qualitätsmetriken basierend auf den Eigenschaften des empfangenen EEG-Signals definiert ist, um die Qualität des bioelektrischen Signals zu messen, wobei die Qualitätsmetriken Folgendes umfassen: Signal-Rausch-Verhältnis (SNR), quadratisches Mittel (RMS) der Amplitude, Kurtosis der Amplitude, Schiefe der Amplitude, Mittelwert der Leistung, Nulldurchgangsrate (ZCR), spektrale Entropie, maximaler Gradient, Autokorrelationsfunktion (ACF), wobei Werte der Qualitätsmetriken die Eigenschaften des bioelektrischen Signals im Frequenz- und Zeitbereich widerspiegeln,
wobei der Satz von Qualitätsmetriken basierend auf Merkmalen konstruiert wird, die die Komplexität und Stärke des Satzes von EEG-Signalen im Zeit- und/oder Frequenzbereich darstellen, wobei die Eigenschaften der empfangenen Vielzahl von bioelektrischen Signalen zusammen mit dem Satz von kontextspezifischen Merkmalen verwendet werden, um die Signalqualität der empfangenen Vielzahl von bioelektrischen Signalen zu bestimmen, wobei die Signalqualität gemäß dem Satz von kontextspezifischen Merkmalen der mobilen Anwendung variiert;
Klassifizieren des ausgewählten Satzes von Elektroden basierend auf dem bewerteten Qualitätsindexwert des Satzes von EEG-Signalen und einem Satz von Artefakten des Satzes von EEG-Signalen, wobei eine kontinuierliche Detektion des Satzes von Artefakten der empfangenen EEG-Signale durchgeführt wird, wobei der Satz von EEG-Signalen als mit dem Satz von Artefakten und ohne Artefakte unter Verwendung von maschinellen Lernalgorithmen gekennzeichnet wird, wobei der Satz von Qualitätsmetriken für jedes Segment normalisiert und einem maschinellen Lernklassifikator zugeführt wird, der dieses bestimmte Segment mit oder ohne Artefakt kennzeichnet; und
Neukalibrieren der Ohren-EEG-Vorrichtung basierend auf dem bewerteten Qualitätsindexwert des Satzes von EEG-Signalen, die von der ausgewählten einen oder den ausgewählten mehreren Elektroden empfangen werden.

6. System (100) nach Anspruch 5, wobei eine Warnung zum Tragen der Ohren-EEG-Vorrichtung an den Benutzer gesendet wird, falls die Aufforderungsnachricht von der mobilen Anwendung nicht vorhanden ist.

7. System (100) nach Anspruch 5, wobei ein Framework erstellt wird, um einen Satz von Qualitätsmetriken für jede der ausgewählten einen oder mehreren Elektroden zu berechnen, die den Satz von EEG-Signalen zu einem Zeitpunkt empfangen, und zu prüfen, ob der Satz von Qualitätsmetriken im Bereich des vordefinierten Schwellenwerts liegt, wobei die vordefinierten Schwellenwerte für den Qualitätsindex anwendungsspezifisch sind.

8. System (100) nach Anspruch 5, wobei:
wenn der Qualitätsindexwert unter einen vordefinierten niedrigsten Schwellenwert für die gegebene mobile Anwendung durch den Benutzer für eine Mindestanzahl von Elektroden fällt, eine EEG-Signalvalidierung und eine Elektrodenauswahl erneut durchgeführt werden; und
wenn der Qualitätsindexwert kleiner als der vordefinierte Schwellenwert ist, die Elektrodenauswahl erneut durchgeführt wird.

9. Ein oder mehrere nichtflüchtige maschinenlesbare Informationsspeichermedien, die eine oder mehrere Anweisungen umfassen, die bei Ausführung durch einen oder mehrere Hardwareprozessoren Folgendes bewirken:
Empfangen, über eine Eingabe/Ausgabe(I/O)-Schnittstelle, einer Vielzahl von bioelektrischen Signalen eines Benutzers von einer Mehrzahl von Elektroden innerhalb einer tragbaren Ohrenzephalographie(EEG)-Vorrichtung, wobei die Mehrzahl von Elektroden eine Masseelektrode und eine Referenzelektrode beinhaltet;
Sammeln eines Satzes von kontextspezifischen Merkmalen von einer mobilen Anwendung innerhalb einer mobilen Vorrichtung, wobei der Satz von kontextspezifischen Merkmalen auf einem anfänglichen Kontext von täglichen Aktivitäten des Benutzers basiert, die automatisch von der tragbaren Ohren-EEG-Vorrichtung gesammelt werden, und der anfängliche Kontext eine Aufforderungsnachricht von der mobilen Anwendung an die Ohren-EEG-Vorrichtung über die mobile Vorrichtung ist, und wobei eine Warnung zum Tragen der Ohren-EEG-Vorrichtung an den Benutzer gesendet wird, falls die Aufforderungsnachricht von der mobilen Anwendung nicht vorhanden ist, wobei der anfängliche Kontext eine Aufforderungsnachricht von der mobilen Anwendung an die tragbare Ohren-EEG-Vorrichtung über die mobile Vorrichtung ist, wobei sich der Kontext auf die Aktivität bezieht, die von dem Benutzer durchgeführt wird;
Bestimmen eines Kontakts jeder der Mehrzahl von Elektroden mit dem Benutzer, um die Vielzahl von bioelektrischen Signalen basierend auf einem Satz von Impedanzwerten zu empfangen, wobei der Satz von Impedanzwerten eine Impedanz zwischen jeder der Mehrzahl von Elektroden und einer Haut-Körper-Schnittstelle des Benutzers ist, und Auswählen eines Satzes von Elektroden mit niedriger Impedanz im Vergleich zu einem vordefinierten Schwellenwert, um die Vielzahl von bioelektrischen Signalen zu empfangen;
Verifizieren der empfangenen Vielzahl von bioelektrischen Signalen von der Mehrzahl von Elektroden basierend auf einer Flachkanaldetektionstechnik, um einen vordefinierten optimalen Spannungspegel aufzuweisen, wobei eine oder mehrere Elektroden der Mehrzahl von Elektroden, die zu der Vielzahl von bioelektrischen Signalen gehören, die einen vordefinierten gesättigten Spannungspegel aufweisen, unter Verwendung der Flachkanaldetektionstechnik entfernt werden;
Auswählen eines Satzes von Elektroden der Mehrzahl von Elektroden, der einen Satz von EEG-Signalen basierend auf einer oder mehreren Eigenschaften der Vielzahl von bioelektrischen Signalen und dem gesammelten Satz von kontextspezifischen Merkmalen empfängt, wobei die eine oder die mehreren Eigenschaften der Vielzahl von bioelektrischen Signalen mindestens eines von (1) im Zeitbereich oder (2) im Frequenzbereich oder (3) sowohl im Zeit- als auch im Frequenzbereich ist, wobei die Auswahltechnik bestimmt, ob die empfangene Vielzahl von bioelektrischen Signalen EEG ist oder nicht, und der ausgewählte Satz von EEG-Signalen von der empfangenen Vielzahl von bioelektrischen Signalen unter Verwendung eines vordefinierten Schwellenspannungspegels basierend auf den Eigenschaften und den kontextspezifischen Merkmalen getrennt wird;
Prüfen des ausgewählten Satzes von Elektroden der Mehrzahl von Elektroden, um eine Warnung an den Benutzer zu senden, um die Ohren-EEG-Vorrichtung neu zu kalibrieren, wenn der ausgewählte Satz von Elektroden kleiner als eine vordefinierte Mindestanzahl von Elektroden ist;
Bewerten eines Qualitätsindexwerts jedes des empfangenen Satzes von EEG-Signalen basierend auf einem vordefinierten Schwellenwert für die mobile Anwendung unter Verwendung eines vortrainierten maschinellen Lernmodells, wobei eine oder mehrere Eigenschaften der empfangenen Vielzahl von bioelektrischen Signalen zusammen mit dem Satz von kontextspezifischen Merkmalen verwendet werden, um die Signalqualität des empfangenen Satzes von EEG-Signalen zu bestimmen, und der Qualitätsindexwert jedes des empfangenen Satzes von EEG-Signalen gemäß den kontextspezifischen Merkmalen der mobilen Anwendung variiert, wobei ein Satz von Qualitätsmetriken basierend auf den Eigenschaften des empfangenen EEG-Signals definiert ist, um die Qualität des bioelektrischen Signals zu messen, wobei die Qualitätsmetriken Folgendes umfassen: Signal-Rausch-Verhältnis (SNR), quadratisches Mittel (RMS) der Amplitude, Kurtosis der Amplitude, Schiefe der Amplitude, Mittelwert der Leistung, Nulldurchgangsrate (ZCR), spektrale Entropie, maximaler Gradient, Autokorrelationsfunktion (ACF), wobei Werte der Qualitätsmetriken die Eigenschaften des bioelektrischen Signals im Frequenz- und Zeitbereich widerspiegeln,
wobei der Satz von Qualitätsmetriken basierend auf Merkmalen konstruiert wird, die die Komplexität und Stärke des Satzes von EEG-Signalen im Zeit- und/oder Frequenzbereich darstellen, wobei die Eigenschaften der empfangenen Vielzahl von bioelektrischen Signalen zusammen mit dem Satz von kontextspezifischen Merkmalen verwendet werden, um die Signalqualität der empfangenen Vielzahl von bioelektrischen Signalen zu bestimmen, wobei die Signalqualität gemäß dem Satz von kontextspezifischen Merkmalen der mobilen Anwendung variiert;
Klassifizieren des ausgewählten Satzes von Elektroden basierend auf dem bewerteten Qualitätsindexwert des Satzes von EEG-Signalen und einem Satz von Artefakten des Satzes von EEG-Signalen, wobei eine kontinuierliche Detektion des Satzes von Artefakten der empfangenen EEG-Signale durchgeführt wird, wobei der Satz von EEG-Signalen als mit dem Satz von Artefakten und ohne Artefakte unter Verwendung von maschinellen Lernalgorithmen gekennzeichnet wird, wobei der Satz von Qualitätsmetriken für jedes Segment normalisiert und einem maschinellen Lernklassifikator zugeführt wird, der dieses bestimmte Segment mit oder ohne Artefakt kennzeichnet; und
Neukalibrieren der Ohren-EEG-Vorrichtung basierend auf dem bewerteten Qualitätsindexwert des Satzes von EEG-Signalen, die von der ausgewählten einen oder den ausgewählten mehreren Elektroden empfangen werden.

10. Ein oder mehrere nichtflüchtige maschinenlesbare Informationsspeichermedien nach Anspruch 9, wobei ein Framework erstellt wird, um einen Satz von Qualitätsmetriken für jede der ausgewählten einen oder mehreren Elektroden zu berechnen, die den Satz von EEG-Signalen zu einem Zeitpunkt empfangen, und zu prüfen, ob der Satz von Qualitätsmetriken im Bereich des vordefinierten Schwellenwerts liegt, und wobei die vordefinierten Schwellenwerte für den Qualitätsindex anwendungsspezifisch sind.

11. Ein oder mehrere nichtflüchtige maschinenlesbare Informationsspeichermedien nach Anspruch 9, wobei:
wenn der Qualitätsindexwert unter einen vordefinierten niedrigsten Schwellenwert für die gegebene mobile Anwendung durch den Benutzer für eine Mindestanzahl von Elektroden fällt, eine EEG-Signalvalidierung und eine Elektrodenauswahl erneut durchgeführt werden; und
wenn der Qualitätsindexwert kleiner als der vordefinierte Schwellenwert ist, die Elektrodenauswahl erneut durchgeführt wird.

## Revendications

1. Procédé mis en œuvre par processeur (300) comprenant :
la réception (302), via une interface d'entrée/sortie (E/S), d'une multitude de signaux bioélectriques d'un utilisateur à partir d'une pluralité d'électrodes dans un dispositif d'électroencéphalographie auriculaire (EEG) portable, dans lequel la pluralité d'électrodes inclut une électrode de masse et une électrode de référence ;
la collecte (304), via un ou plusieurs processeurs matériels, d'un ensemble de caractéristiques spécifiques au contexte à partir d'une application mobile dans un dispositif mobile, dans lequel l'ensemble de caractéristiques spécifiques au contexte est basé sur un contexte initial à partir d'activités quotidiennes de l'utilisateur collectées automatiquement à partir du dispositif d'EEG auriculaire portable, dans lequel le contexte initial est un message d'invite de l'application mobile au dispositif d'EEG auriculaire portable via le dispositif mobile, le contexte se rapportant à l'activité effectuée par l'utilisateur ;
la détermination (306), via les un ou plusieurs processeurs matériels, d'un contact de chacune de la pluralité d'électrodes avec l'utilisateur afin de recevoir la multitude de signaux bioélectriques sur la base d'un ensemble de valeurs d'impédance, dans lequel l'ensemble de valeurs d'impédance est une impédance entre chacune de la pluralité d'électrodes et une interface peau-corps de l'utilisateur, et la sélection d'un ensemble d'électrodes avec une faible impédance par rapport à un seuil prédéfini afin de recevoir la multitude de signaux bioélectriques ;
la vérification (308), via les un ou plusieurs processeurs matériels, de la multitude reçue de signaux bioélectriques à partir de la pluralité d'électrodes sur la base d'une technique de détection de canal plat pour avoir un niveau de tension optimal prédéfini, dans lequel une ou plusieurs électrodes de la pluralité d'électrodes sont retirées en fonction la multitude de signaux bioélectriques ayant un niveau de tension saturé prédéfini en utilisant la technique de détection de canal plat ;
la sélection (310), via les un ou plusieurs processeurs matériels, d'un ensemble d'électrodes de la pluralité d'électrodes qui reçoit un ensemble de signaux EEG sur la base d'une ou plusieurs caractéristiques de la multitude de signaux bioélectriques et de l'ensemble collecté de caractéristiques spécifiques au contexte, dans lequel les une ou plusieurs caractéristiques de la multitude de signaux bioélectriques sont au moins l'une parmi (1) dans le domaine temporel, ou (2) dans le domaine fréquentiel, ou (3) à la fois dans le domaine temporel et fréquentiel, dans lequel la technique de sélection détermine si la multitude reçue de signaux bioélectriques est EEG ou non et l'ensemble sélectionné de signaux EEG est séparé de la multitude reçue de signaux bioélectriques en utilisant un niveau de tension seuil prédéfini sur la base des caractéristiques et des caractéristiques spécifiques au contexte ;
la vérification (312), via les un ou plusieurs processeurs matériels, de l'ensemble sélectionné d'électrodes de la pluralité d'électrodes pour envoyer une alerte à l'utilisateur pour réétalonner le dispositif d'EEG auriculaire si l'ensemble sélectionné d'électrodes est inférieur à un nombre minimum prédéfini d'électrodes ;
l'évaluation (314), via les un ou plusieurs processeurs matériels, d'une valeur d'indice de qualité de chacun de l'ensemble reçu de signaux EEG sur la base d'une valeur seuil prédéfinie pour l'application mobile en utilisant un modèle d'apprentissage machine pré-entraîné, dans lequel une ou plusieurs caractéristiques de la multitude reçue de signaux bioélectriques conjointement avec l'ensemble de caractéristiques spécifiques au contexte sont utilisées pour déterminer la qualité de signal de l'ensemble reçu de signaux EEG, dans lequel un ensemble de métriques de qualité sur la base des caractéristiques du signal EEG reçu est défini pour mesurer la qualité du signal bioélectrique, dans lequel les métriques de qualité comprennent : un rapport signal sur bruit (SNR), une moyenne quadratique (RMS) de l'amplitude, un kurtosis de l'amplitude, une asymétrie de l'amplitude, une puissance moyenne, un taux de passage par zéro (ZCR), une entropie spectrale, un gradient maximum, une fonction d'autocorrélation (ACF), dans lequel des valeurs des métriques de qualité reflètent les caractéristiques du signal bioélectrique dans le domaine fréquentiel et temporel,
dans lequel l'ensemble de métriques de qualité est construit sur la base de caractéristiques qui représentent une complexité et la force de l'ensemble de signaux EEG dans le domaine temporel et/ou fréquentiel, dans lequel les caractéristiques de la multitude reçue de signaux bioélectriques conjointement avec l'ensemble de caractéristiques spécifiques au contexte sont utilisées pour déterminer la qualité de signal de la multitude reçue de signaux bioélectriques, dans lequel la qualité de signal varie selon l'ensemble de caractéristiques spécifiques au contexte de l'application mobile ;
la classification (316), via les un ou plusieurs processeurs matériels, de l'ensemble sélectionné d'électrodes sur la base de la valeur d'indice de qualité évaluée de l'ensemble de signaux EEG et d'un ensemble d'artefacts de l'ensemble de signaux EEG, dans lequel une détection continue de l'ensemble d'artefacts des signaux EEG reçus est effectuée, dans lequel l'ensemble de signaux EEG sont étiquetés comme présentant des artefacts ou ne présentant pas d'artefacts en utilisant des algorithmes d'apprentissage machine, dans lequel l'ensemble de métriques de qualité pour chaque segment est normalisé et fourni à un classificateur d'apprentissage machine qui étiquette ce segment particulier avec ou sans artefact ; et
le réétalonnage (318), via les un ou plusieurs processeurs matériels, du dispositif d'EEG auriculaire sur la base de la valeur d'indice de qualité évaluée de l'ensemble de signaux EEG reçus à partir des une ou plusieurs électrodes sélectionnées.

2. Procédé mis en œuvre par processeur (300) selon la revendication 1, dans lequel une alerte pour porter le dispositif d'EEG auriculaire est envoyée à l'utilisateur en cas d'absence du message d'invite de l'application mobile.

3. Procédé mis en œuvre par processeur (300) selon la revendication 1, dans lequel une infrastructure est créée pour calculer un ensemble de métriques de qualité pour chacune des une ou plusieurs électrodes sélectionnées recevant l'ensemble de signaux EEG à un instant et vérifier si l'ensemble de métriques de qualité est dans la plage de la valeur seuil prédéfinie, et dans lequel les valeurs seuils prédéfinies pour l'indice de qualité sont spécifiques à une application.

4. Procédé mis en œuvre par processeur (300) selon la revendication 1, dans lequel :
si la valeur d'indice de qualité chute en dessous d'un seuil le plus bas prédéfini pour l'application mobile donnée par l'utilisateur pour un nombre minimum d'électrodes, une validation de signal EEG et une sélection d'électrode sont effectuées à nouveau ; et
si la valeur d'indice de qualité est inférieure à la valeur seuil prédéfinie, la sélection d'électrode est effectuée à nouveau.

5. Système (100) comprenant :
une mémoire (110) stockant des instructions ;
une ou plusieurs interfaces d'entrée/sortie (E/S) (104) ; et
un ou plusieurs processeurs matériels (108) couplés à la mémoire (110) via les une ou plusieurs interfaces E/S (104), dans lequel les un ou plusieurs processeurs matériels (108) sont configurés par les instructions pour :
recevoir une multitude de signaux bioélectriques d'un utilisateur à partir d'une pluralité d'électrodes dans un dispositif d'électroencéphalographie auriculaire (EEG) portable, dans lequel la pluralité d'électrodes inclut une électrode de masse et une électrode de référence ;
collecter un ensemble de caractéristiques spécifiques au contexte à partir d'une application mobile dans un dispositif mobile, dans lequel l'ensemble de caractéristiques spécifiques au contexte est basé sur un contexte initial à partir d'activités quotidiennes de l'utilisateur collectées automatiquement à partir du dispositif d'EEG auriculaire portable, dans lequel le contexte initial est un message d'invite de l'application mobile au dispositif d'EEG auriculaire portable via le dispositif mobile, le contexte se rapportant à l'activité effectuée par l'utilisateur ;
déterminer un contact de chacune de la pluralité d'électrodes avec l'utilisateur afin de recevoir la multitude de signaux bioélectriques sur la base d'un ensemble de valeurs d'impédance, dans lequel l'ensemble de valeurs d'impédance est une impédance entre chacune de la pluralité d'électrodes et une interface peau-corps de l'utilisateur, et sélectionner un ensemble d'électrodes avec une faible impédance par rapport à un seuil prédéfini afin de recevoir la multitude de signaux bioélectriques ;
vérifier la multitude reçue de signaux bioélectriques à partir de la pluralité d'électrodes sur la base d'une technique de détection de canal plat pour avoir un niveau de tension optimal prédéfini, dans lequel une ou plusieurs électrodes de la pluralité d'électrodes sont retirées correspondant à la multitude de signaux bioélectriques ayant un niveau de tension saturé prédéfini en utilisant la technique de détection de canal plat ;
sélectionner un ensemble d'électrodes de la pluralité d'électrodes qui reçoit un ensemble de signaux EEG sur la base d'une ou plusieurs caractéristiques de la multitude de signaux bioélectriques et de l'ensemble collecté de caractéristiques spécifiques au contexte, dans lequel les une ou plusieurs caractéristiques de la multitude de signaux bioélectriques sont au moins l'une parmi (1) dans le domaine temporel, ou (2) dans le domaine fréquentiel, ou (3) à la fois dans le domaine temporel et fréquentiel, dans lequel la technique de sélection détermine si la multitude reçue de signaux bioélectriques est EEG ou non et l'ensemble sélectionné de signaux EEG est séparé de la multitude reçue de signaux bioélectriques en utilisant un niveau de tension seuil prédéfini sur la base des caractéristiques et des caractéristiques spécifiques au contexte ;
vérifier l'ensemble sélectionné d'électrodes de la pluralité d'électrodes pour envoyer une alerte à l'utilisateur pour réétalonner le dispositif d'EEG auriculaire si l'ensemble sélectionné d'électrodes est inférieur à un nombre minimum prédéfini d'électrodes ;
évaluer une valeur d'indice de qualité de chacun de l'ensemble reçu de signaux EEG sur la base d'une valeur seuil prédéfinie pour l'application mobile en utilisant un modèle d'apprentissage machine pré-entraîné, dans lequel une ou plusieurs caractéristiques de la multitude reçue de signaux bioélectriques conjointement avec l'ensemble de caractéristiques spécifiques au contexte sont utilisées pour déterminer la qualité de signal de l'ensemble reçu de signaux EEG, dans lequel un ensemble de métriques de qualité sur la base des caractéristiques du signal EEG reçu est défini pour mesurer la qualité du signal bioélectrique, dans lequel les métriques de qualité comprennent : un rapport signal sur bruit (SNR), une moyenne quadratique (RMS) de l'amplitude, un kurtosis de l'amplitude, une asymétrie de l'amplitude, une moyenne de la puissance, un taux de passage par zéro (ZCR), une entropie spectrale, un gradient maximum, une fonction d'autocorrélation (ACF), dans lequel des valeurs des métriques de qualité reflètent les caractéristiques du signal bioélectrique dans le domaine fréquentiel et temporel,
dans lequel l'ensemble de métriques de qualité est construit sur la base de caractéristiques qui représentent la complexité et la force de l'ensemble de signaux EEG dans le domaine temporel et/ou fréquentiel, dans lequel les caractéristiques de la multitude reçue de signaux bioélectriques conjointement avec l'ensemble de caractéristiques spécifiques au contexte sont utilisées pour déterminer la qualité de signal de la multitude reçue de signaux bioélectriques, dans lequel la qualité de signal varie selon l'ensemble de caractéristiques spécifiques au contexte de l'application mobile ;
classifier l'ensemble sélectionné d'électrodes sur la base de la valeur d'indice de qualité évaluée de l'ensemble de signaux EEG et d'un ensemble d'artefacts de l'ensemble de signaux EEG, dans lequel une détection continue de l'ensemble d'artefacts des signaux EEG reçus est effectuée, dans lequel l'ensemble de signaux EEG sont étiquetés comme présentant des artefacts ou ne présentant pas d'artefacts en utilisant des algorithmes d'apprentissage machine, dans lequel l'ensemble de métriques de qualité pour chaque segment est normalisé et fourni à un classificateur d'apprentissage machine qui étiquette ce segment particulier avec ou sans artefact ; et
réétalonner le dispositif d'EEG auriculaire sur la base de la valeur d'indice de qualité évaluée de l'ensemble de signaux EEG reçus à partir des une ou plusieurs électrodes sélectionnées.

6. Système (100) selon la revendication 5, dans lequel une alerte pour porter le dispositif d'EEG auriculaire est envoyée à l'utilisateur en cas d'absence du message d'invite de l'application mobile.

7. Système (100) selon la revendication 5, dans lequel une infrastructure est créée pour calculer un ensemble de métriques de qualité pour chacune des une ou plusieurs électrodes sélectionnées recevant l'ensemble de signaux EEG à un instant et vérifier si l'ensemble de métriques de qualité est dans la plage de la valeur seuil prédéfinie, dans lequel les valeurs seuils prédéfinies pour l'indice de qualité sont spécifiques à une application.

8. Système (100) selon la revendication 5, dans lequel :
si la valeur d'indice de qualité chute en dessous d'un seuil le plus bas prédéfini pour l'application mobile donnée par l'utilisateur pour un nombre minimum d'électrodes, une validation de signal EEG et une sélection d'électrode sont effectuées à nouveau ; et
si la valeur d'indice de qualité est inférieure à la valeur seuil prédéfinie, la sélection d'électrode est effectuée à nouveau.

9. Un ou plusieurs supports de stockage d'informations lisibles par machine non transitoires comprenant une ou plusieurs instructions qui, lorsqu'elles sont exécutées par un ou plusieurs processeurs matériels, amènent :
la réception, via une interface d'entrée/sortie (E/S), d'une multitude de signaux bioélectriques d'un utilisateur à partir d'une pluralité d'électrodes dans un dispositif d'électroencéphalographie auriculaire (EEG) portable, dans lequel la pluralité d'électrodes inclut une électrode de masse et une électrode de référence ;
la collecte d'un ensemble de caractéristiques spécifiques au contexte à partir d'une application mobile dans un dispositif mobile, dans lequel l'ensemble de caractéristiques spécifiques au contexte est basé sur un contexte initial à partir d'activités quotidiennes de l'utilisateur collectées automatiquement à partir du dispositif d'EEG auriculaire portable et le contexte initial est un message d'invite de l'application mobile au dispositif d'EEG auriculaire via le dispositif mobile, et dans lequel une alerte pour porter le dispositif d'EEG auriculaire est envoyée à l'utilisateur en cas d'absence du message d'invite de l'application mobile, dans lequel le contexte initial est un message d'invite de l'application mobile au dispositif d'EEG auriculaire portable via le dispositif mobile, le contexte se rapportant à l'activité effectuée par l'utilisateur ;
la détermination d'un contact de chacune de la pluralité d'électrodes avec l'utilisateur afin de recevoir la multitude de signaux bioélectriques sur la base d'un ensemble de valeurs d'impédance, dans lequel l'ensemble de valeurs d'impédance est une impédance entre chacune de la pluralité d'électrodes et une interface peau-corps de l'utilisateur et la sélection d'un ensemble d'électrodes avec une faible impédance par rapport à un seuil prédéfini afin de recevoir la multitude de signaux bioélectriques ;
la vérification de la multitude reçue de signaux bioélectriques à partir de la pluralité d'électrodes sur la base d'une technique de détection de canal plat pour avoir un niveau de tension optimal prédéfini, dans lequel une ou plusieurs électrodes de la pluralité d'électrodes sont retirées en fonction de la multitude de signaux bioélectriques ayant un niveau de tension saturé prédéfini en utilisant la technique de détection de canal plat ;
la sélection d'un ensemble d'électrodes de la pluralité d'électrodes qui reçoit un ensemble de signaux EEG sur la base d'une ou plusieurs caractéristiques de la multitude de signaux bioélectriques et de l'ensemble collecté de caractéristiques spécifiques au contexte, dans lequel les une ou plusieurs caractéristiques de la multitude de signaux bioélectriques sont au moins l'une parmi (1) dans le domaine temporel, ou (2) dans le domaine fréquentiel, ou (3) à la fois dans le domaine temporel et fréquentiel, dans lequel la technique de sélection détermine si la multitude reçue de signaux bioélectriques est EEG ou non et l'ensemble sélectionné de signaux EEG est séparé de la multitude reçue de signaux bioélectriques en utilisant un niveau de tension seuil prédéfini sur la base des caractéristiques et des caractéristiques spécifiques au contexte ;
la vérification de l'ensemble sélectionné d'électrodes de la pluralité d'électrodes pour envoyer une alerte à l'utilisateur pour réétalonner le dispositif d'EEG auriculaire si l'ensemble sélectionné d'électrodes est inférieur à un nombre minimum prédéfini d'électrodes ;
l'évaluation d'une valeur d'indice de qualité de chacun de l'ensemble reçu de signaux EEG sur la base d'une valeur seuil prédéfinie pour l'application mobile en utilisant un modèle d'apprentissage machine pré-entraîné, dans lequel une ou plusieurs caractéristiques de la multitude reçue de signaux bioélectriques conjointement avec l'ensemble de caractéristiques spécifiques au contexte sont utilisées pour déterminer la qualité de signal de l'ensemble reçu de signaux EEG, et la valeur d'indice de qualité de chacun de l'ensemble reçu de signaux EEG varie selon les caractéristiques spécifiques au contexte de l'application mobile, dans lequel un ensemble de métriques de qualité sur la base des caractéristiques du signal EEG reçu est défini pour mesurer la qualité du signal bioélectrique, dans lequel les métriques de qualité comprennent : un rapport signal sur bruit (SNR), une moyenne quadratique (RMS) de l'amplitude, un kurtosis de l'amplitude, une asymétrie de l'amplitude, une moyenne de la puissance, un taux de passage par zéro (ZCR), une entropie spectrale, un gradient maximum, une fonction d'autocorrélation (ACF), dans lequel des valeurs des métriques de qualité reflètent les caractéristiques du signal bioélectrique dans le domaine fréquentiel et temporel,
dans lequel l'ensemble de métriques de qualité est construit sur la base de caractéristiques qui représentent la complexité et la force de l'ensemble de signaux EEG dans le domaine temporel et/ou fréquentiel, dans lequel les caractéristiques de la multitude reçue de signaux bioélectriques conjointement avec l'ensemble de caractéristiques spécifiques au contexte sont utilisées pour déterminer la qualité de signal de la multitude reçue de signaux bioélectriques, dans lequel la qualité de signal varie selon l'ensemble de caractéristiques spécifiques au contexte de l'application mobile ;
la classification de l'ensemble sélectionné d'électrodes sur la base de la valeur d'indice de qualité évaluée de l'ensemble de signaux EEG et d'un ensemble d'artefacts de l'ensemble de signaux EEG, dans lequel une détection continue de l'ensemble d'artefacts des signaux EEG reçus est effectuée, dans lequel l'ensemble de signaux EEG sont étiquetés comme présentant des artefacts ou ne présentant pas d'artefacts en utilisant des algorithmes d'apprentissage machine, dans lequel l'ensemble de métriques de qualité pour chaque segment est normalisé et fourni à un classificateur d'apprentissage machine qui étiquette ce segment particulier avec ou sans artefact ; et
le réétalonnage du dispositif d'EEG auriculaire sur la base de la valeur d'indice de qualité évaluée de l'ensemble de signaux EEG reçus à partir des une ou plusieurs électrodes sélectionnées.

10. Un ou plusieurs supports de stockage d'informations lisibles par machine non transitoires selon la revendication 9, dans lequel une infrastructure est créée pour calculer un ensemble de métriques de qualité pour chacune des une ou plusieurs électrodes sélectionnées recevant l'ensemble de signaux EEG à un instant et vérifier si l'ensemble de métriques de qualité est dans la plage de la valeur seuil prédéfinie, et dans lequel les valeurs seuils prédéfinies pour l'indice de qualité sont spécifiques à une application.

11. Un ou plusieurs supports de stockage d'informations lisibles par machine non transitoires selon la revendication 9, dans lequel :
si la valeur d'indice de qualité chute en dessous d'un seuil le plus bas prédéfini pour l'application mobile donnée par l'utilisateur pour un nombre minimum d'électrodes, une validation de signal EEG et une sélection d'électrode sont effectuées à nouveau ; et
si la valeur d'indice de qualité est inférieure à la valeur seuil prédéfinie, la sélection d'électrode est effectuée à nouveau.
